# EUROPEAN PATENT APPLICATION

(11) **EP 4 553 504 A1**
(43) Date of publication of application: **14.05.2025**
(21) Application number: 23835181.1
(22) Date of filing: 26.05.2023
(51) Int. Cl.: G01N 35/00

(54) **AUTOMATED ANALYSIS SYSTEM AND ALARM MANAGEMENT METHOD**

(30) Priority: 07.07.2022 JP 2022109975
(71) Applicant: HITACHI HIGH-TECH CORPORATION, Tokyo 105-6409 (JP)
(72) Inventor: SEKI, Yoshihiro, Tokyo 105-6409 (JP)
(74) Representative: Strehl & Partner mbB
(86) International application number: PCT/JP2023/019736
(87) International publication number: WO 2024/009645

(57) **Abstract**

The present invention facilitates identification of the cause of an alarm and specification of a method for addressing the alarm. This automated analysis system comprises an inspection processing unit that executes at least one process for the purpose of inspection, and a management device that manages the inspection processing unit. The management device is provided with: an alarm detection unit that detects that an alarm indicating the occurrence of an abnormality in the inspection processing unit is being emitted; an alarm notification unit that, in accordance with the result of detection by the alarm detection unit, issues a notification to indicate that the alarm is being emitted; and a related alarm determination unit that determines, from among related alarms that are other alarms having a prescribed degree of relatedness to the alarm being emitted, a related alarm that is in an unaddressed state.

## Description

### Technical Field

The present disclosure relates to an automated analysis system and an alarm management method for analyzing biological samples such as blood and urine.

### Background Art

An automated analysis device that performs an analysis on a biological sample such as blood and urine is a device indispensable in modern diagnostics due to advantages such as high reproducibility of analysis accuracy and rapid analysis processing. There is a plurality of types of such an automated analysis device depending on types of analysis (for example, a colorimetric analysis device that performs a biochemical analysis, an immune analysis device that analyzes antigens or antibodies in a sample using antigen-antibody reactions, a coagulation analysis device that measures blood's clotting ability, and blood cell counters that measure the number of blood cell components in blood).

When analyzing the sample with the automated analysis device, a pretreatment device that executes a pretreatment such as centrifugation of blood or aliquoting of the sample to prepare a plurality of aliquot specimens is provided alongside the above-described automated analysis device, and implements an automated analysis system together with it. It is common for a single hospital or testing center to use a plurality of these automated analysis devices and pretreatment devices of the sample. In addition, the automated analysis system may also be provided with a robot that operates in a test room to assist a test technician in performing a duty thereof and a storage device for storing a specimen and a reagent. In this way, in the automated analysis system, various peripheral devices are included in addition to the automated analysis device, and they integrally operate as a whole.

When analyzing the sample with such an automated analysis system, an abnormality may occur in the automated analysis device, the pretreatment device, the robot, the storage device, or the like in the system, and an alarm may be generated to notify the abnormality. In this case, if the alarm is not quickly handled, the analysis may be delayed and a diagnosis of a doctor may be delayed. As a technique for handling such a problem, PTL 1 proposes an automated analysis system in which operation information of each of devices is collected by a management device independent of the devices, a cause of an alarm is estimated by collating the collected operation information with a known abnormality pattern, and alarm information and a plurality of cause candidates are notified.

However, in an alarm notification method in PTL 1, when an abnormality occurs in one device in the system, a terminal of the test technician is notified of alarm information of the one device. With only the notification of the alarm information, the test technician or the like cannot know a specific cause of the generated alarm and a handling method thereof. The test technician who receives the alarm notification determines the specific cause of the generated alarm and an appropriate handling method according to the knowledge thereof and an operation history in the system. However, when the knowledge of the test technician or information on the operation history is not sufficient, it may be difficult to make the determination, and it may take time for the determination.

### Citation List

### Patent Literature

PTL 1: JP2010-266271A

### Summary of Invention

### Technical Problem

The present disclosure provides an automated analysis system and an alarm management method that facilitate identification of a cause and a handling method of an alarm.

### Solution to Problem

An automated analysis system according to the present disclosure is an automated analysis system including a test processing unit configured to execute at least a part of processing for a test of a specimen and a management device configured to manage the test processing unit. The management device includes an alarm detection unit configured to detect that an alarm indicating occurrence of an abnormality in the test processing unit is generated, an alarm notification unit configured to notify, according to a detection result of the alarm detection unit, that the alarm is generated, and a related alarm determination unit configured to determine, among related alarms that are other alarms each having a predetermined degree of relevance in relation to the alarm, a related alarm that is in an unhandled state.

### Advantageous Effects of Invention

According to the present disclosure, it is possible to provide an automated analysis system and an alarm management method that facilitate identification of a cause of an alarm and a handling method.

### Brief Description of Drawings

[FIG. 1] FIG. 1 is a block diagram illustrating a schematic configuration of an automated analysis system according to an embodiment.
[FIG. 2] FIG. 2 is a diagram illustrating an example of an alarm management table 110.
[FIG. 3] FIG. 3 is a diagram illustrating an example of a data structure of a related alarm management table 111.
[FIG. 4] FIG. 4 is a diagram illustrating an example of an alarm list screen 201 that is notified by an alarm notification unit 107 and displayed on a display terminal 116.
[FIG. 5] FIG. 5 is a diagram illustrating an example of an alarm detailed information screen 301.
[FIG. 6] FIG. 6 illustrates an alarm detailed information screen 501 that is an example of an alarm detailed information screen displaying in-test-room related alarm information.
[FIG. 7] FIG. 7 illustrates an alarm detailed information screen 401 that is an example of an alarm detailed information screen displaying in-device related alarm information (analysis-related).

### Description of Embodiments

Hereinafter, an embodiment will be described with reference to the accompanying drawings. In the accompanying drawings, functionally the same elements may be denoted by the same reference numerals. Although the accompanying drawings illustrate embodiments and examples according to the principle of the present disclosure, the accompanying drawings are for understanding the present disclosure and are not intended to interpret the present disclosure in a limited manner. The description of the present specification is merely a typical example and does not limit the scope of claims or application examples of the present disclosure in any sense.

Embodiments are described in sufficient detail for those skilled in the art to implement the present disclosure, but it should be understood that other implementations and aspects are possible, and changes in configurations and structures and replacement of various elements are possible without departing from the scope and spirit of the technical idea of the present disclosure. Therefore, the following description should not be interpreted as being limited thereto.

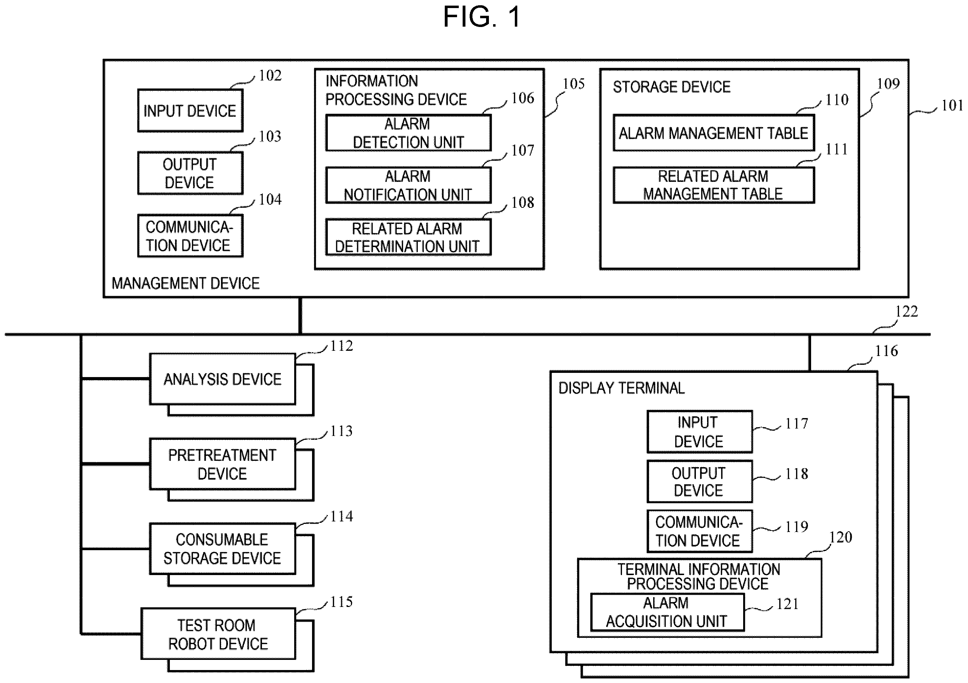

A schematic configuration of an automated analysis system according to the embodiment will be described with reference to a block diagram of FIG. 1. For example, the automated analysis system is configured such that a management device 101, a plurality of analysis devices 112, a plurality of pretreatment devices 113, a plurality of consumable storage devices 114, a plurality of test room robot devices 115, and a display terminal 116 (a display device) are connected via a network 122, and data communication therebetween is possible. The plurality of analysis devices 112 are analysis devices that perform analyses on a specimen, and the plurality of pretreatment devices 113, the plurality of consumable storage devices 114, and the plurality of test room robot devices 115 function as peripheral devices for assisting operations of the analysis device 112. The analysis devices 112 and the peripheral devices constitute an analysis processing unit, and the management device 101 manages the analysis processing unit. The devices 112 to 115 may be provided in one test room or may be provided in a plurality of test rooms. The management device 101 does not need to manage all of the devices 112 to 115 and may manage a device (referred to as a "test processing unit" in the present description) that executes at least a part of the processing for test.

The display terminal 116 is a display device that displays an analysis result by the analysis device 112 (an analysis processing unit) and receives information related to operation states of the analysis device 112, the pretreatment device 113, the consumable storage device 114, and the test room robot device 115 to display the information. The network 122 is a network that connects various devices and terminals, and may be a wired/wireless local area network, a wired/wireless global area network, a mobile telephone network, or a combination thereof.

The analysis device 112 is a device that automatically executes an analysis of various components in the specimen after the specimen is supplied and a reagent or the like is input. The pretreatment device 113 is a device that automatically executes a pretreatment for executing a clinical test, is connected to the analysis device 112, and has a function of transporting the specimen to the analysis device 112 after the pretreatment. The pretreatment device 113 is also connected to the consumable storage device 114 and may also have a function of supplying a reagent and a consumable required for the analysis to the analysis device 112.

The consumable storage device 114 is a device that stores the reagent and the consumable used in the analysis device 112 and/or the specimen and may have a refrigerating function or a freezing function for storing the reagent and the specimen at a suitable temperature. The test room robot device 115 is a robot for assisting a test technician in performing work thereof in the test room by, for example, executing works such as transportation, mixing, and disposal of the specimen and the reagent in the test room. Specifically, a wide range of works are executed, such as a sorting work of an analysis target specimen, a reagent replacement work in the analysis device, and a consumable replacement work in the analysis device 112 or the pretreatment device 113. In the illustrated example, the devices 112 to 115 are provided in plural, respectively, and the number thereof is not limited to a specific number and may be one. The analysis device 112 may operate alone or may operate in combination of a plurality of analysis devices.

The display terminal 116 is a display device for executing display of an operating status of the automated analysis system, an alarm generation status, and the like and includes an input device 117, an output device 118, a communication device 119, and a terminal information processing device 120. The display terminal 116 may be, for example, a mobile terminal, a wearable terminal, or a glasses-type terminal carried by the test technician, or may be a notebook type or desktop type PC or a digital signage provided in the test room. By using a portable terminal as the display terminal 116, the test technician can view the alarm information even at a remote place. When a digital signage is used as the display terminal 116, a plurality of test technicians can simultaneously view the alarm information. In addition, the display terminal 116 may be substituted by an operation or information display PC provided in the management device 101, the analysis device 112, the pretreatment device 113, or the consumable storage device 114, or an operation or information display PC in the test room robot device 115.

The input device 117 is a device that processes inputs of various types of data and signals including data related to a result of an analysis in the analysis device 112, an alarm from the management device 101, and the like. The output device 118 is, for example, a display, a printer, or the like, and is a device for outputting various types of data. The communication device 119 is a communication unit that performs data communication with an external device including a management device. The terminal information processing device 120 is an information processing unit for processing various types of information input via the input device 117 or the like, and includes an alarm acquisition unit 121 that acquires the alarm information.

The management device 101 has a function of managing the plurality of analysis devices 112, the plurality of pretreatment devices 113, the plurality of consumable storage devices 114, and the plurality of test room robot devices 115, and determining abnormalities in management data, particularly in the devices 112 to 115, notifying alarms, and managing the alarm information. Specifically, the management device 101 includes, for example, an input device 102, an output device 103, a communication device 104, an information processing device 105, and a storage device 109. The management device 101 can collect the alarm information of the various devices 112 to 115 via the network 122 to manage the alarm information across a plurality of types of devices. The management device 101 may be provided as a local server within the test room to prevent information from being taken outside for purposes such as protecting personal information, or may be provided as a cloud server outside the test room for a provider of the automated analysis systems to manage information across a plurality of test rooms.

In addition to a keyboard and a mouse, the input device 102 includes, for example, an input device using a screen touch panel of the output device 103 and may also be used as the output device 103. As the input device 102, a device capable of performing an input operation by voice, gaze, and finger operations, such as a voice recognition engine, a gaze dynamic measurement device, and a finger dynamic measurement device, may be adopted.

The output device 103 is a monitor that displays input information or other result information and may be substituted by the display terminal 116. The communication device 104 is a device for the management device 101 to communicate with at least one of the plurality of analysis devices 112, the plurality of pretreatment devices 113, the plurality of consumable storage devices 114, the plurality of test room robot devices 115, and the display terminal 116 via the network 122, and may use a wired/wireless local area network, a wired/wireless global area network, a mobile phone network, or a combination thereof.

The communication device 104 may have a function of performing encrypting and decrypting of communication contents, and may perform encrypted communication with the analysis device 112, the pretreatment device 113, the consumable storage device 114, the test room robot device 115, and the display terminal 116. Particularly, in the communication between the management device 101 and the analysis device 112, the pretreatment device 113, the consumable storage device 114, the test room robot device 115, and the display terminal 116, encrypted communication using a common key encryption scheme is performed to be able to prevent problems such as spoofing of communication and interception of communication.

The information processing device 105 is a computer for executing functions required for the management device 101 to perform the alarm notification and further includes an alarm detection unit 106, an alarm notification unit 107, and a related alarm determination unit 108.

The alarm detection unit 106 has a function of receiving, via the communication device 104, an alarm indicating an abnormality occurring in various devices including the analysis device 112, the pretreatment device 113, the consumable storage device 114, and the test room robot device 115, and detecting that an alarm is generated in these devices.

The alarm notification unit 107 has a function of transmitting the received alarm information and information of the related alarm calculated by the related alarm determination unit 108 to the display terminal 115 via the communication device 104. The alarm detection unit 106 and the alarm notification unit 107 may have a function of performing encrypting and decrypting of the communication contents separately from the function of performing the encrypting and decrypting of the communication contents owned by the communication device 104. In particular, in the communication between the management device 101 and the analysis device 112, the pretreatment device 113, the consumable storage device 114, the test room robot device 115, and the display terminal 116, by performing encrypted communication when handling information should be kept confidential such as personal information, it can be expected to be effective in preventing problems such as personal information leakage. The related alarm determination unit 108 has a function of determining a related alarm in an unhandled state among related alarms which are other alarms having a predetermined degree of relevance in relation to the alarm detected by the alarm detection unit 106. By determining a related alarm that is related to a newly generated alarm and is in an unhandled state, it becomes easy to identify a specific cause of the generated alarm and a handling method thereof. Alarm notification methods by the alarm notification unit 107 include transmission to the display terminal 116 as described above, as well as email delivery, voice notification, and video notification using AR/VR, and are not limited to a specific method.

The storage device 109 is a storage device that stores programs and data executed by the information processing device 105 and can use, for example, a non-volatile memory such as an EEPROM or a flash memory, an HDD, or an SSD. The storage device 109 includes an alarm management table 110 and a related alarm management table 111. The alarm management table 110 is a table for managing and storing contents of an alarm that is generated. The related alarm management table 111 is a table for managing the alarm that is generated and a related alarm, which is related to the generated alarm, is another generated alarm, and is unhandled.

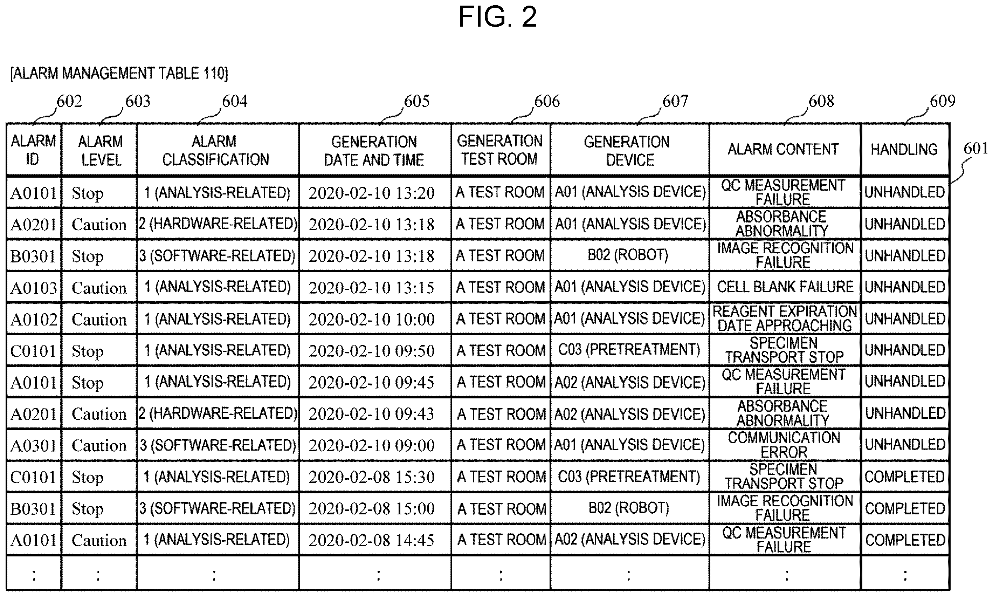

An example of the alarm management table 110 will be described with reference to FIG. 2.

FIG. 2 illustrates the example of the alarm management table 110. The management device 101 registers the alarm information acquired by the alarm detection unit 106 in the alarm management table 110 and manages it. The acquisition of the alarm information by the alarm detection unit 106 may be a case of acquiring the alarm information notified of events from the various devices 112 to 115, a case of transmitting a request of the alarm information acquisition from the management device 101 to the various devices 112 to 115 and acquiring a response thereof, or a case of a combination thereof.

The alarm management table 110 includes a management table 601 including columns 602 to 609, and information in the columns 602 to 609 of one row in a row direction indicates information about one alarm. The alarm ID column 602 describes an alarm ID for identifying an alarm. The alarm level column 603 describes an alarm level indicating a degree of importance of the alarm. For example, when the alarm level is "Stop", it means that an alarm causing a target device to stop is generated and indicates that the degree of importance is large. When the alarm level is "Caution", it indicates that the alarm does not reach device stop, and in that sense, the degree of importance is not higher than that of Stop, but a certain degree of caution is required. In the illustrated example, the alarm level is only Stop and Caution, and of course, other alarm levels may be set.

The alarm classification column 604 describes alarm classification information for classifying alarms by types (abnormality occurrence location, type of abnormality, or the like). For example, "1" of the alarm classification means that an alarm (an analysis-related alarm) related to an analysis operation is generated, such as an abnormality of a reagent or a consumable used for the clinical test, an abnormality of calibration, and an abnormality of QC measurement. "2" of the alarm classification means that a (hardware-related) alarm related to a hardware configuration itself of the analysis devices 112 to 115 is generated. "3" of the alarm classification means that an alarm related to a software abnormality (occurrence of a bug, crash, or the like) of the analysis devices 112 to 115 is generated. In addition to the examples exemplified here, the alarm classification may include various classifications such as ISE-related, which focuses on an ion concentration to perform the measurement, immunoassay-related, which involves immune item measurement, and biochemical measurement-related, which involves biochemical item measurement, and is not limited to the examples described above.

The generation date and time column 605 describes information related to date and time when the alarm is generated. For example, the information "2020-02-10 13: 20" means that the alarm was generated at 13:20 on Feb. 10, 2020. The generation test room column 606 describes information for identifying a test room in which the alarm is generated. When only one test room is provided and all the devices are provided in one test room, the column 606 may be omitted from the alarm management table 110.

The generation device column 607 is a column including an identification symbol of a device in which the alarm is generated. For example, the identification symbol "A01" means that the alarm is generated in the analysis device 112 having an identification symbol A01. The alarm content column 608 is a column including a content of the generated alarm. For example, when the alarm content is "QC measurement failure", it means that an alarm is generated due to a failure in the QC measurement performed for reagent accuracy management. The handling column 609 includes information indicating whether handling for the generated alarm is completed or whether the alarm is in a state still requiring handling (unhandled yet). For example, "unhandled" means a state in which handling of the alarm is not performed (not completed) and the handling is required. "Completed" means that the handling of the alarm is completed, and no new handling is required.

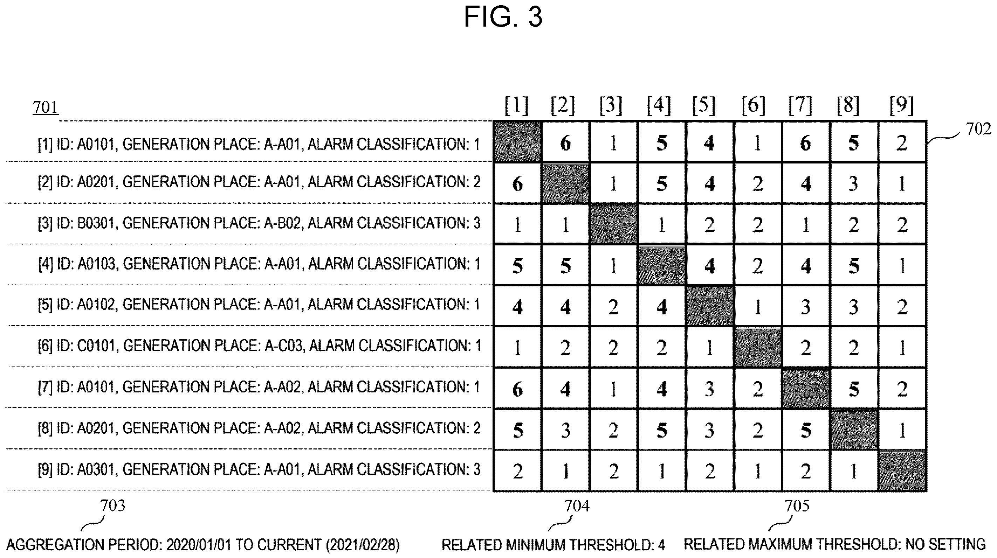

FIG. 3 illustrates an example of a data structure of the related alarm management table 111. The related alarm management table 111 includes a related alarm aggregation table 702, and may include an aggregation period 703, a related minimum threshold 704, and a related maximum threshold 705 as setting items.

Based on information about the related alarm management table 111, the related alarm determination unit 108 of the management device 101 determines another alarm that is related to the newly generated alarm and is unhandled as the related alarm. The related alarm management table 111 illustrates a result obtained by calculating a degree of relevance of a plurality of alarms in the related alarm aggregation table 702. Specifically, the related alarm aggregation table 702 aggregates the number of times of duplicate generations between the plurality of alarms within a period set in the aggregation period 703.

In the related alarm aggregation table 702, all combinations that may occur concerning the ID and a generation place of the alarm and the alarm classification are illustrated in a two-dimensional table, and the number of times of duplicate generations is managed by numerical values at crossing portions of a matrix of the two-dimensional table. In the example illustrated in FIG. 3, for the sake of simplicity, only an alarm whose handing is "unhandled" in FIG. 2 is displayed, but in the actual related alarm aggregation table 702, not only an unhandled alarm but also a handled alarm can be included in the related alarm aggregation table 702.

An item 701 in a row direction and a column direction of the related alarm aggregation table 702 indicates an alarm that may occur, and the numerical values (1, 2, 3,...) at the crossing portions indicate the number of times of duplicate generations, which is the number of times different alarms occur duplicately in the same period or related periods. For example, the row and column of [1] mean an alarm whose alarm ID is A0101, generation place is A-A01 (an A01 device in an A test room), and alarm classification is 1 (analysis-related). The numerical values of the crossing portions between the row and the column (for example, the numerical values 6, 1, 5, 4, 1, 6, 5, and 2 in the first row) indicate the number of times of duplicate generations with another alarm. As to be described later, data about the number of times of duplicate generations is updated (accumulatively added) at a timing at which the related alarm is specified in the related alarm determination unit 108 for one alarm.

The aggregation period 703 is a setting item in which an aggregation start period and an aggregation end period can be set. When the aggregation period 703 is not set, the aggregation is performed in the entire period. For example, when the aggregation period 703 is "2020-01-01-current", it means that the aggregation start period is set to 2020-01-01 and the aggregation end period is set to the current date (2021-02-28).

The related minimum threshold 704 is a minimum threshold for determining the related alarm. When a value of the number of times of duplicate generations aggregated in the related alarm aggregation table 702 is less than a value set in the related minimum threshold 704, the corresponding two alarms are determined to be unrelated alarms.

The related maximum threshold 705 is a maximum threshold for determining the related alarm. When the value of the number of times of duplicate generations aggregated in the related alarm aggregation table 702 is larger than a value set in the related maximum threshold 705, the corresponding two alarms are determined to be unrelated alarms. By setting the related maximum threshold 705, when the number of times of duplicate generations is excessively large, it becomes possible to exclude the alarm from the related alarms since the reliability of the numerical value is low. In this way, in the present embodiment, it is determined whether an alarm is the related alarm according to a magnitude relation between the minimum threshold and/or the maximum threshold and the number of times of duplicate generations.

When the related minimum threshold 704 or the related maximum threshold 705 is not set, it is determined that the larger the numerical value is, the higher the degree of relevance is without performing the above-described exclusion operation, and it is determined whether an alarm is a related alarm. The aggregation period 703, the related minimum threshold 704, and the related maximum threshold 705 can be set by an authorized facility manager, a maintenance company staff, a test technician, or other operators among operators who operate the management device 101.

The aggregation period 703, the related minimum threshold 704, and the related maximum threshold 705 may be received from the management device 101 in the related alarm management table 111 as illustrated in FIG. 3, but instead of (or in addition to) this, they may be input by the alarm acquisition unit 121 of the display terminal 116. In this case, the management device 101 creates the related alarm management table 111 based on a value set in the display terminal 116, and returns the alarm information to the display terminal 116 in which the value is set. In the related alarm aggregation table 702, a table in which the number of times of duplicate generations between alarms within a set period is aggregated is exemplified. In addition, it is possible to aggregate the degree of relevance by calculating a degree of similarity based on cluster analysis. Alternatively, the degree of relevance can be obtained by learning the degree of similarity by a method such as deep learning.

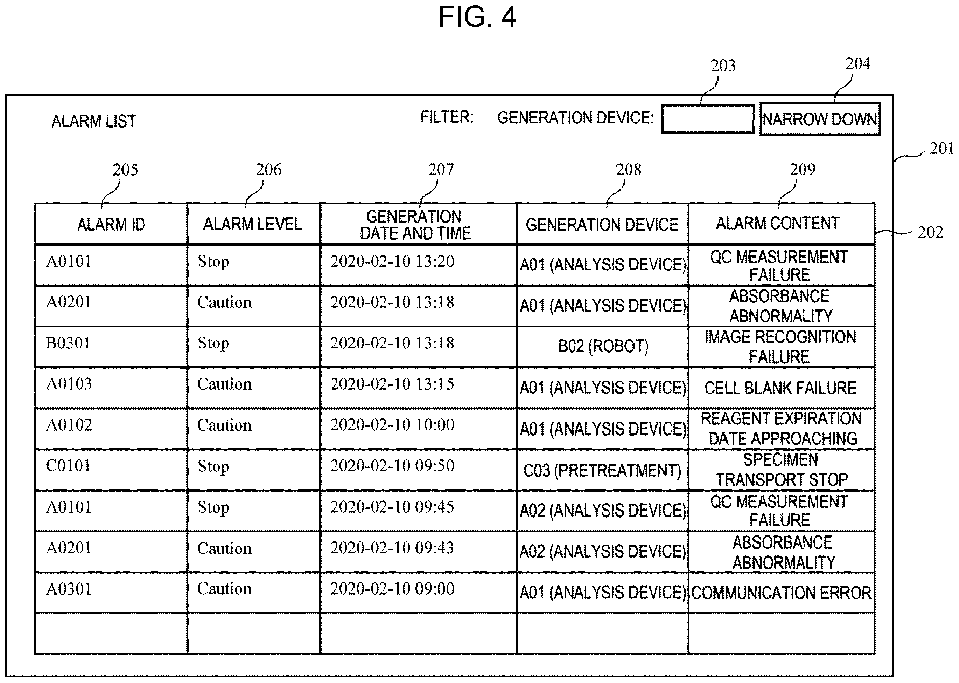

FIG. 4 illustrates an example of an alarm list screen 201 that is notified by the alarm notification unit 107 and is displayed on the display terminal 116. The alarm list screen 201 exemplified here displays alarms that are not handled among the alarms that have already occurred. The alarm list screen 201 may include, for example, an alarm list table 202 and may further include an input column 203 and a "narrow-down" button 204 used for filtering the displayed alarms.

The input column 203 is, for example, an input column for filtering the alarm by a name or the identification symbol of a device in which the alarm occurs. For example, the test technician can update the display of the alarm list table 202 by inputting the identification symbol "A01" as a generation device by which a narrow-down is desired to be performed in the input column 203 and pressing the "narrow-down" button 204. The test technician can confirm the alarm list table in which A01 is displayed as the generation device. In addition to or instead of the input column 203 for inputting the identification symbol of the device, an input column in which other conditions such as the alarm ID, the alarm level, the generation date and time, and the alarm content can be input may be included.

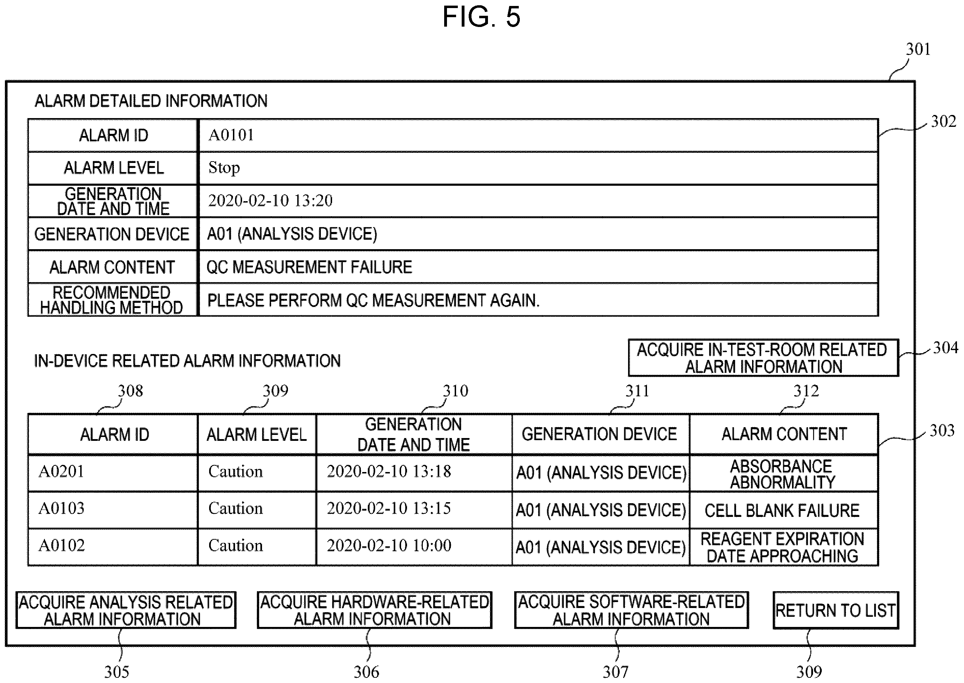

The alarm list table 202 is a display example of table format information in which information about an alarm that is currently occurring or has already occurred but is not handled can be confirmed in a list. The alarm list table 202 illustrated in FIG. 4 includes an alarm ID column 205, an alarm level column 206, a generation date and time column 207, a generation device column 208, and an alarm content column 209 as examples of display items. An information group arranged in a row direction indicates contents of one alarm that is generated. By selecting the alarm information of one row by clicking on the screen, it is possible to transition to a detailed information screen of the alarm. For example, when the test technician selects, using a mouse, alarm information at the top of the alarm list table 202, in which the alarm ID is A0101, the alarm level is Stop, the generation date and time is 13:20 on Feb. 10, 2020, the generation device is A01 (analysis device), and the alarm content is QC measurement failure, the screen transitions to an alarm detailed information screen 301 as illustrated in FIG. 5. When the alarm occurs, an operation mode in which the detailed information screen 301 of the alarm as illustrated in FIG. 5 is automatically displayed without a selection operation can also be set.

The column 205 to the column 209 in the alarm list table 202 may function as buttons, and any one of the column 205 to the column 209 may be selected by double-clicking a mouse to sort the information in that column in an ascending order/descending order, such as a dictionary order or a date order. In the alarm list table 202, when there is a large number of pieces of alarm information and they cannot be displayed on one screen, the list 202 may be scrolled to display the alarm information.

FIG. 5 is an example of the alarm detailed information screen 301. The alarm detailed information screen 301 may be automatically displayed on the display terminal 116 when a new alarm is generated from the alarm notification unit 107 of the management device 101, or may be displayed when a specific alarm is selected on the alarm list screen 201 in FIG. 4. The alarm detailed information screen is transmitted from the alarm notification unit 107 to the display terminal 116 via the communication device 104 and the network 122.

The screen in FIG. 5 is an example and may include elements other than illustrated those, and a part of elements may be omitted. The alarm detailed information screen 301 includes, for example, an alarm detailed information table 302, an in-device related alarm information table 303, an in-test-room related alarm information acquisition button 304, an analysis related alarm information acquisition button 305, a hardware related alarm information acquisition button 306, and a software related alarm information acquisition button 307. The buttons 304 to 307 are buttons to be pressed when information different from information being displayed is acquired and a transition to another screen is performed.

The alarm detailed information table 302 is information in a table format in which detailed information on the selected alarm is collected. In addition to the information displayed in the alarm list table 202, a recommended handling method may be displayed, for example as illustrated in FIG. 5.

In addition, the in-device related alarm information table 303 can display a related alarm in the same device as that of a new alarm among the related alarms that are related to the alarm newly notified by the alarm notification unit 107 and are unhandled. Displays (308 to 312) of the in-device related alarm information table 303 are controlled according to the information in the related alarm management table 111. The in-device related alarm information table 303 can display a related alarm in the same device as that of a new alarm, and as to be described later, by operating the buttons 304 to 307, the related alarm to be displayed can be switched as appropriate.

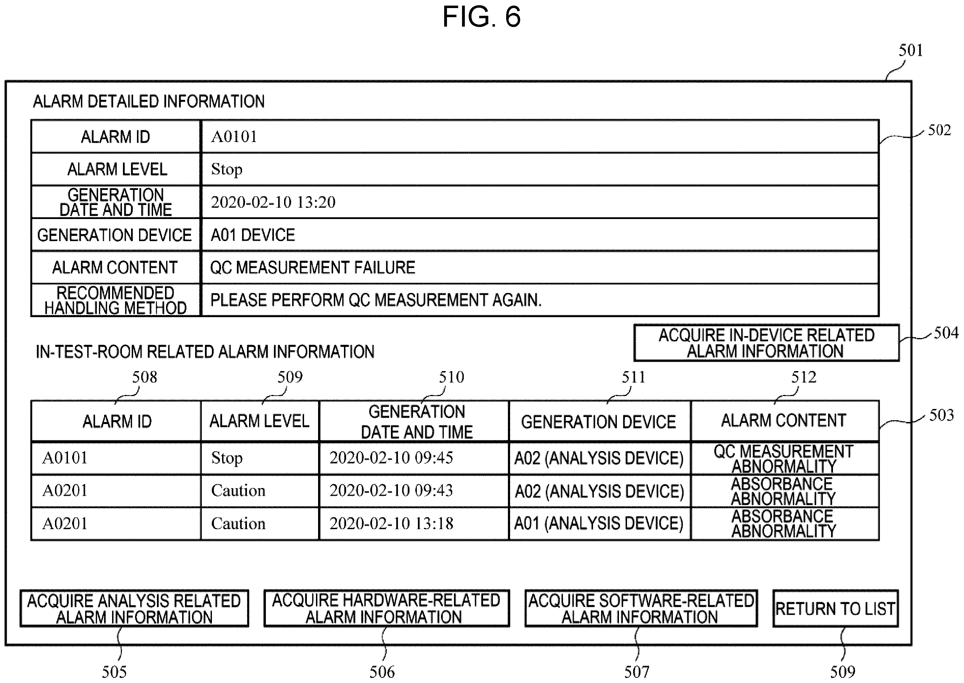

The in-test-room related alarm information acquisition button 304 is pressed on the screen in FIG. 5, leading to a transition to an alarm detailed information screen (see FIG. 6) displaying in-test-room related alarm information for displaying a related alarm related to a device present in the same test room as the device related to a newly generated alarm among the related alarms. Since the related alarm related to the device present in the same test room is displayed, it is possible to easily identify a cause of the generation of the alarm in relation to the device in the same test room.

FIG. 6 illustrates an alarm detailed information screen 501, which is an example of the alarm detailed information screen displaying the in-test-room related alarm information. The alarm detailed information screen 501 displaying the in-test-room related alarm information includes, for example, an alarm detailed information table 502 and an in-test-room related alarm information table 503. In addition, an in-device related alarm information acquisition button 504, an analysis related alarm information acquisition button 505, a hardware related alarm information acquisition button 506, and a software related alarm information acquisition button 507 for a transition to another screen can be included.

Similarly to the alarm detailed information table 302, the alarm detailed information table 502 is information in a table format in which detailed information related to the alarm is collected. For example, an alarm content and a recommended handling method may additionally be included.

The in-test-room related alarm information table 503 is information in a table format in which information on a related alarm that is currently generating or is already generated in the same test room as that of the newly notified alarm and is unhandled is collected. A column 508 to a column 512 display an alarm ID, an alarm level, a generation date and time, a generation device, and an alarm content, respectively.

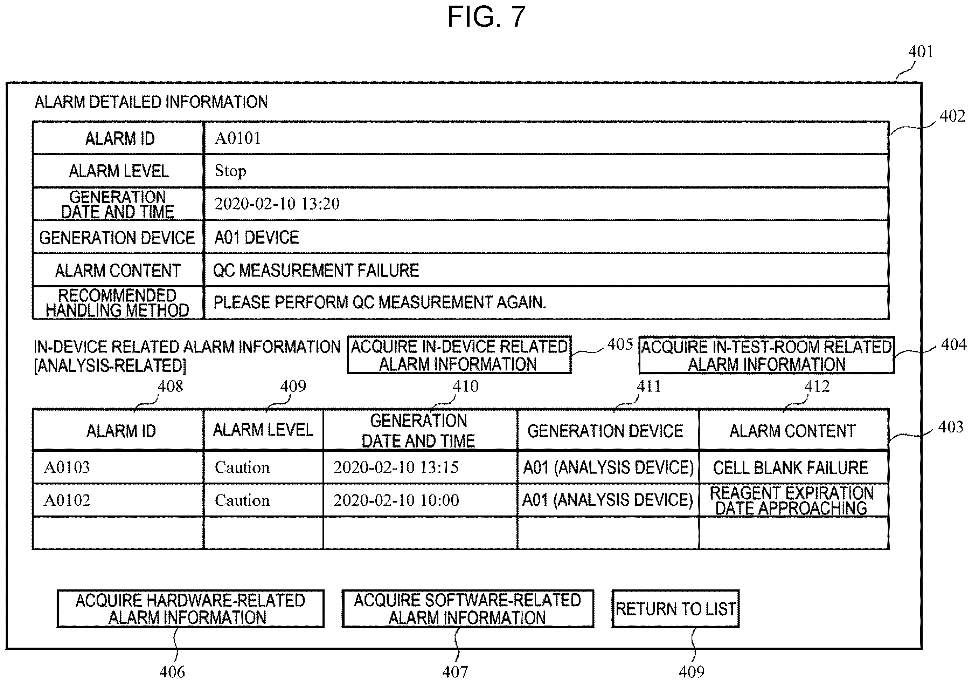

In contrast, the analysis related alarm information acquisition button 305 is pressed on the screen in FIG. 5, leading to a transition to an alarm detailed information screen (see FIG. 7) displaying analysis related alarm information for displaying a related alarm that is in the same device as the analysis device 112 related to the newly generated alarm and is related to the same analysis operation among the related alarms. Since the related alarm related to the same analysis operation is displayed, it may be easy to identify the specific cause of the generation of the new alarm. An alarm detailed information screen 401 in FIG. 7 is an example of the alarm detailed information screen displaying the analysis related alarm information among in-device related alarms, and similarly to FIG. 5, includes an alarm detailed information table 402 as well as an in-device related alarm information table 403. In addition, for example, an in-test-room related alarm information acquisition button 404, an in-device related alarm information acquisition button 405, a hardware related alarm information acquisition button 406, and a software related alarm information acquisition button 407 are included as buttons for a screen transition.

The in-device related alarm information table 403 is information in a table format in which information on an alarm that is generated currently in the same device as that of the newly generated alarm, is unhandled, is classified as analysis-related in an alarm type, and has a high degree of relevance is collected. A column 408 to a column 412 display an alarm ID, an alarm level, a generation date and time, a generation device, and an alarm content, respectively.

Even in the alarm detailed information screens in FIGS. 6 and 7, a button for a transition to another alarm detailed information screen can be displayed, and the display of the detailed information screen can be switched between FIGS. 5, 6, and 7 by pressing the button. In addition, by pressing "return to list" buttons 309, 409, or 509, it is possible to return to the alarm list screen 201 in FIG. 4.

When the hardware related alarm information acquisition buttons 506 and 406 are pressed, it is possible to transition to an alarm detailed information screen in which related alarm information related to hardware among related alarms is displayed. When a newly generated alarm is related to hardware, since related alarm information related to hardware is similarly displayed, it may be easy to identify a specific cause of the generation of the new alarm.

When the software related alarm information acquisition buttons 507 and 407 are pressed, it is possible to transition to an alarm detailed information screen in which related alarm information related to software among related alarms is displayed. When a newly generated alarm is related to software, since related alarm information related to software is similarly displayed, it may be easy to identify a specific cause of the generation of the new alarm.

A specific example of operations of the automated analysis system according to the present embodiment will be described below. Here, for example, a case will be described in which the A01 device, which is one of the analysis devices 112, fails in QC measurement, and therefore the device operation stops.

One of the analysis devices 112 (A01 device) failing in the QC measurement and stopping the device operation generates an alarm to notify a QC measurement failure. Information on the generated alarm is transmitted to the management device 101 via the network 122. However, the information on the generated alarm may be stored in the analysis device 112 (A01 device) that generates the alarm to display the alarm by one monitor of the device or for the purpose of log analysis.

To facilitate communication between devices, the alarm notification via the network 122 may be notified to the pretreatment device 113, the consumable storage device 114, and the test room robot device 115, or may be notified directly to the display terminal 116 held by the test technician when there is no management device 101 in the test room.

When the alarm information is transmitted to the network 122 by the analysis device 112, the management device 101 acquires a communication message by the communication device 104 and decodes the alarm information by the alarm detection unit 106 in the information processing device 105. After decoding the alarm information, the management device 101 registers the acquired alarm information of the QC measurement failure in the alarm management table 110 in the storage device 109. The alarm information is registered in the alarm management table 110 in a format as illustrated in FIG. 2.

For example, as in the case of the alarm ID = A0101 of the top row in FIG. 2, it is registered that an alarm related to the A01 device provided in the A test room was generated on 2020-02-10, 13:20. In the alarm classification of the column 604, "1" indicating that the alarm is an analysis related alarm is registered. The alarm classification information can be added to the acquired alarm information by the management device 101, or the alarm classification information can be included in the notified alarm information from the beginning. The handling column 609 also indicates that the handling of the acquired alarm of the QC measurement failure is not completed.

After the alarm management table 110 is updated, the management device 101 operates the related alarm determination unit 108 to update the related alarm management table 111. In the related alarm aggregation table 702 of the related alarm management table 111, since the generated alarm of the QC measurement failure is generated in the A01 device of the A test room, the generation place is "A-A01", and the row and column of [1] correspond to this alarm.

The related alarm management table 111 may be updated in the following manner. That is, the update can be performed by adding 1 to the number of times of duplicate generations with the generated alarm for an alarm for which the handling column 609 is "unhandled" in the alarm management table 110 within the period set in the aggregation period 703 when the generated alarm information is acquired by the management device 101. In the related alarm management table 111 illustrated in FIG. 3, since only unhandled alarms are displayed, for example, values at the crossing portions of the [1] alarm row and the [1] alarm column can be added by 1.

After the related alarm management table 111 is updated, the management device 101 transmits, by the alarm notification unit 107, the alarm information to the display terminal 116 via the network 122 connected with the communication device 104. The alarm notification method by the alarm notification unit 107 includes: (1) a case in which the test technician selects an alarm from the alarm list screen 201 on the display terminal 116 and notifies the selected alarm information, and (2) a case in which the information of the updated row is notified when the related alarm management table 111 is updated. For example, in the case of the alarm notification of the former (1), the test technician selects the alarm information desired to be seen in detail in the alarm list screen 201 of FIG. 4 and displays the alarm detailed information screen 301. In contrast, in the latter case (2), the alarm detailed information screen 301 is notified from the management device 101 to the display terminal 116 for the information of the row [1] updated in the related alarm management table 111. Here, the former alarm notification will be described.

To confirm the alarm information, the test technician confirms the alarm list screen 201 on the display terminal 116. In response to an instruction from the test technician, the display terminal 116 requests, by the alarm acquisition unit 121, the management device 101 to transmit information required for drawing the alarm list screen 201 via the network 122 to which the communication device 119 is connected.

In response to a request for the information required for drawing the alarm list screen 201, the management device 101 returns, by the communication device 104, a list of alarms for which the handling column of the column 609 is unhandled from the alarm management table 110 as a response via the network 122. Here, the alarm information included in the returned response may include information in the column 609 that is "completed" based on a request from the display terminal 116. In this case, the alarm information included in the response may be only alarm information in which the handling column 609 is "completed". The display terminal 116 acquires, by the alarm acquisition unit 121, the response from the management device 101 via the network 122 to which the communication device 119 is connected and draws the alarm list screen 201 on the screen.

The test technician confirms the alarm list screen 201 displayed on the display terminal 116 used by the test technician, and can know that, for example, an alarm of "2020-02-10 13:20", "A01", and "QC measurement failure" is generated. In this case, to know details of this alarm, the test technician can select a corresponding row on the alarm list screen 201 and shift to the alarm detailed information screen.

In response to an instruction from the test technician, the display terminal 116 transmits, by the alarm acquisition unit 121, a request to transmit information required for drawing the alarm detailed information screen 301 to the management device 101 via the network 122 to which the communication device 119 is connected. In response thereto, the management device 101 refers to the alarm management table 110 and transmits the required information to the display terminal 116. Specifically, according to the request from the display terminal 116, the alarm management table 110 is analyzed, and alarm information in which the alarm ID is A0101, the alarm level is Stop, the generation date and time is 2020-02-10, 13:20, the generation device is A01, and the alarm content is QC measurement failure is identified and is transmitted to the display terminal 116 as the information for drawing the alarm detailed information screen. In addition, the related alarm management table 111 is referred to according to the specified alarm information, and the related alarm information corresponding to the specified alarm information is identified.

When the related alarm information is identified, the related minimum threshold 704 and the related maximum threshold 705 are also referred to, and alarm information satisfying them is extracted as the related alarm information. For example, if the identified alarm information is alarm information [1] in FIG. 3 and the related minimum threshold is set to [3], alarm information [2], [4], [5], [7], and [8] are selected as the related alarms.

When there is a setting for displaying the in-device related alarm as the related alarm, the management device 101 refers to the generation device column 607, extracts only the alarm generated in the same device as the selected alarm information [1], and displays the alarm in the in-device related alarm information table 303 as the in-device related alarm.

The test technician may confirm the alarm detailed information screen 301 and the in-device related alarm information table 303 and may notice that alarms of an absorbance abnormality, a cell blank failure, and reagent expiration date approaching are generated. In this case, the test technician can press the analysis related alarm information acquisition button 305 to acquire (extract) the analysis related alarm information as the related alarm information.

When the analysis related alarm information acquisition button 305 is pressed, the management device 101 refers to the alarm management table 110 and the related alarm management table 111 to extract alarm information common in analysis related items in the alarm content column 608 as the analysis related alarm information. The extracted analysis related alarm information may be displayed in the in-device related alarm information table 403 as illustrated in FIG. 7.

The test technician may confirm the alarm detailed information screen 401 as illustrated in FIG. 7 and may notice that alarms of a "cell blank failure" or "reagent expiration date approaching" are generated as the analysis related alarms among the in-device related alarms. In this case, the test technician can suspect that a specific cause of the alarm is deterioration of a measurement cell and deterioration of the reagent.

The deterioration of the measurement cell may cause an absorbance abnormality of a measurement sensor or an abnormal test result due to deterioration of a test cell inside the device, which is a consumable, and a change in a wall surface state. In contrast, the deterioration of the reagent may change a state of compounds in the reagent, potentially causing problems such as a more difficult reaction than usual during a test or occurrence of unexpected reactions.

The test technician can confirm when an analysis device cell is replaced because an expiration date of the reagent is approaching but the reagent is still within the expiration date. As a result, it can be understood that a considerable amount of time has passed since the last cell replacement, and it can be determined that the specific cause of the alarm is the deterioration of the measurement cell and that a handling method is to replace the measurement cell. In this case, it is useful for the test technician to also confirm the related alarms in the test room to confirm whether other devices in the test room have similar problems.

When the in-test-room related alarm information acquisition button 404 is pressed on the alarm detailed information screen 401, a corresponding request is transmitted to the management device 101. The management device 101 can refer to the alarm management table 110 and the related alarm management table 111, extract the alarm information related to the device in the same test room as the related alarm information by referring to the generation device column 607, and display the related alarm information in the in-test-room related alarm information table 503.

Various embodiments of the present disclosure have been described above, but the present disclosure is not limited to the embodiments described above and includes various modifications. For example, the embodiments described above are described in detail to describe the present disclosure in an easy-to-understand manner and are not necessarily limited to including all the described configurations. Apart of a configuration of a certain embodiment can be replaced with a configuration of another embodiment, and a configuration of another embodiment can also be added to a configuration of a certain embodiment. In addition, another configuration can be added to, deleted from, or replaced with a part of a configuration of each embodiment.

### Reference Signs List

101: management device
102: input device
103: output device
104: communication device
105: information processing device
106: alarm detection unit
107: alarm notification unit
108: related alarm determination unit
109: storage device
110: alarm management table
111: related alarm management table
112: analysis device
113: pretreatment device
114: consumable storage device
115: test room robot device
116: display terminal
117: input device
118: output device
119: communication device
120: terminal information processing device
121: alarm acquisition unit
122: network

## Claims

1. An automated analysis system comprising:
a test processing unit configured to execute at least a part of processing for a test of a specimen; and
a management device configured to manage the test processing unit, wherein
the management device includes
an alarm detection unit configured to detect that an alarm indicating an occurrence of an abnormality in the test processing unit is generated,
an alarm notification unit configured to notify, according to a detection result of the alarm detection unit, that the alarm is generated, and
a related alarm determination unit configured to determine, among related alarms that are other alarms each having a predetermined degree of relevance in relation to the alarm, a related alarm that is in an unhandled state.

2. The automated analysis system according to claim 1, wherein
the management device further includes an alarm management table for managing the generated alarm, and
the alarm management table includes information indicating whether the alarm is handled or unhandled.

3. The automated analysis system according to claim 1, wherein
the alarm notification unit is configured to notify alarm detailed information related to details of the alarm and the related alarm.

4. The automated analysis system according to claim 1, wherein
the test processing unit includes an analysis device configured to perform an analysis of the specimen and a peripheral device thereof, and
the peripheral device includes at least one or more of
a pretreatment device configured to perform a pretreatment of the specimen,
a consumable storage device configured to store a consumable including a reagent, and
a robot device configured to operate in a test room.

5. The automated analysis system according to claim 1, wherein
the related alarm determination unit is configured to determine the degree of relevance based on the number of times of duplicate generations between the alarm and the related alarm.

6. The automated analysis system according to claim 5, wherein
the related alarm determination unit is configured to determine the degree of relevance based on the number of times of duplicate generations within a predetermined period.

7. The automated analysis system according to claim 5, wherein
the related alarm determination unit determines the degree of relevance based on a magnitude relationship between the number of times of duplicate generations and a predetermined threshold.

8. The automated analysis system according to claim 1, wherein
the related alarm determination unit determines, as the related alarm, an alarm related to a device the same as a device for which the alarm is generated.

9. The automated analysis system according to claim 1, wherein
the related alarm determination unit determines, as the related alarm, an alarm related to another device provided in a test room the same as a test room provided with a device for which the alarm is generated.

10. The automated analysis system according to claim 1, wherein
the related alarm determination unit determines an alarm related to an abnormality of software as the related alarm.

11. The automated analysis system according to claim 1, wherein
the related alarm determination unit determines an alarm related to an abnormality of hardware as the related alarm.

12. The automated analysis system according to claim 1, further comprising:
a display device configured to display an analysis result and an operation state in the test processing unit, wherein
the display device is configured to display a related alarm information table that displays an alarm determined to be the related alarm by the related alarm determination unit, and the related alarm information table is configured to select and display a related alarm having a predetermined characteristic among the related alarms.

13. An alarm management method for an automated analysis system, the alarm management method comprising:
a step of detecting that an alarm indicating an occurrence of an abnormality in the automated analysis system is generated;
a step of notifying, according to a result of the detection, that the alarm is generated; and
a step of determining, among related alarms that are other alarms having a predetermined degree of relevance in relation to the alarm, a related alarm that is in an unhandled state.

14. The alarm management method according to claim 13, wherein
in the step of determining the related alarm, the degree of relevance is determined based on the number of times of duplicate generations between the alarm and the related alarm.

15. The alarm management method according to claim 14, wherein
in the step of determining the related alarm, the degree of relevance is determined based on the number of times of duplicate generations within a predetermined period.

16. The alarm management method according to claim 14, wherein
in the step of determining the related alarm, the degree of relevance is determined based on a magnitude relationship between the number of times of duplicate generations and a predetermined threshold.
